# EUROPEAN PATENT APPLICATION

(11) **EP 2 481 283 A2**
(43) Date of publication of application: **01.08.2012**
(21) Application number: 10817439.2
(22) Date of filing: 17.09.2010
(51) Int. Cl.: A01M 7/00, B05B 7/12, B05B 1/30

(54) **ULTRA-LOW VOLUME SPRAYING DEVICE**

(30) Priority: 18.09.2009 KR 20090088481; 20.11.2009 KR 20090112322
(71) Applicant: Wui, Sung Soo, Seoul 132-742 (KR)
(72) Inventor: Wui, Sung Soo, Seoul 132-742 (KR)
(74) Representative: von Kreisler Selting Werner
(86) International application number: PCT/KR2010/006399
(87) International publication number: WO 2011/034370

(57) **Abstract**

The present invention proposes to solve the abovementioned problems, and it is an aim of the present invention to provide an ultra-low volume spraying device which can spray chemicals under optimum spraying conditions in accordance with the characteristics of the chemicals being sprayed in the form of ultra-fine particles.

## Description

### [Technical Field]

The present invention relates to an ultra-low volume spraying device able to spray chemicals in the form of ultrafine particles so as to improve drawbacks of an atomizing type spraying device which sprays the chemicals diluted with a large amount of water, and more particularly to an ultra-low volume spraying device capable of allowing spraying conditions of chemicals to be automatically set up using characteristic information of the chemicals, which is encoded and recorded in a chemical container, and enabling the chemicals to be sprayed under optimum spraying conditions taking into consideration characteristics of each chemical even if a user does not take additional actions.

The present invention also relates to an ultra-low volume spraying device capable of preventing blockage of a spray nozzle, which is often developed in the ultra-low volume spraying device, and allowing characteristic information of chemicals, which is stored in respective chemical containers, and spraying information, which actually sprays the chemicals, to be read and stored in a control unit, thereby enabling objective data such as types of the chemicals sprayed and sprayed time and date to be utilized in an agricultural product traceability system, a farming daily record, or the like.

### [Background Art]

In general, a conventional high volume (HV) atomizing type spraying device is constituted in such a manner that liquid state agrochemicals or fertilizers (hereinafter, referred to as 'chemicals') made of toxic substances harmful to a human body and environment are diluted with a large amount of water and directly sprayed onto targeted pests and the surface of crops.

Accordingly, spraying highly toxic and harmful chemicals over a wide area using the atomizing type spraying device requires preparation in which after the chemicals are opened and poured into a mixing tank, the chemicals are properly diluted with a metered amount of water by a user.

In such preparation, however, the conventional atomizing type spraying device has a problem in that when a user opens harmful chemicals container cap or spills the chemicals by mistake, a user's respiratory system or skin is directly exposed to the the high concentrated harmful chemicals, thereby damaging a user's health. There is also a problem in that chemical injury or deterioration in chemical efficacy is caused by inaccurate metering.

In the case of using the atomizing type spraying device in a greenhouse, spraying operations of chemicals are very difficult due to unventilated, hot and high humidity conditions. There is also a problem in that a user runs into poisoning danger by toxicity of chemicals.

As a spraying method using the conventional atomizing type spraying device sprays chemicals, up to 80 percent of the total pesticide applied to the targets on the plants eventually reach the soil because of large particle size, thereby contamination of the siol in this manner has caused majer changes in the population of non-target organisms. Consequently, the spraying method has a problem of causing sustainable cultivation failure due to soil contamination. In addition, once the soil is contaminated, the contaminated soil may not be naturally rehabilitate by rain unlike the outdoors because plants are consistently cultivated, particularly in the case of a greenhouse, thereby further increasing the problem.

Furthermore, since chemicals developed in recent years tend to gradually narrow a biological spectrum taking into consideration influence on a human body and environment (namely, high toxicity is exhibited only on particular disease and insect pests, whereas chemical efficacy is not exhibited on other disease and insect pests), a large sprayed amount of water increases greenhouse humidity together with sprayed chemicals for the particular disease and insect pests in the case of using the atomizing type spraying device, thereby having a problem of providing good conditions for the survival of other disease and insect pests.

To solve such problems, recently an ultra-low volume spraying device is mainly utilized to spray chemicals containing high concentrations of active ingredients to confined area in the form of ultrafine particles and to float the deoplets in the space for a long time, thereby allowing chemical to be reached to the target. The ultra-low volume spraying device has several advantages in that labor costs are reduced, a user is not exposed to harmful agrochemicals, and it is not necessary to perform a secondary process (for example, dilution) of chemicals, because of easy automatic pest control. Accordingly, it may be possible to improve convenience and accuracy for use and to facilitate standardization.

In addition, since most chemicals sprayed in the form of ultrafine particles fall onto plant surfaces or soil surfaces but a sprayed amount of chemicals is very small, the sprayed chemicals are naturally decomposed by ultraviolet light without flowing or infiltrating into the soil, thereby preventing soil contamination. Furthermore, unlike the atomizing type spraying device, since the ultra-low volume spraying device does not use a large amount of water, the device may be considerably decreased in size and cost.

In order to maximize delivery and control efficacy in the case of the ultra-low volume spraying device according to the related art, spraying particle size must be properly adjusted depending on characteristics of chemicals such as viscosity and volatility, but it may be nearly impossible to distinguish the spraying particle size sprayed in the form of ultrafine particles with the naked eye and to optimally adjust the same. Therefore, there is a problem in that it is difficult to spray the chemicals under the optimum conditions taking into consideration characteristics of each chemical.

Also, in the case of the ultra-low volume spraying device according to the related art, there is a problem in that blockage of a spraying nozzle is often generated due to a carbonization phenomenon occurring during heating and spraying of chemicals having high viscosity because of the very small size of the spraying nozzle.

### [Disclosure]

### [Technical Problem]

Therefore, the present invention has been made in view of the above problems, and it is an object of the present invention to provide an ultra-low volume spraying device capable of enabling the chemicals to be sprayed in the form of ultrafine particles under optimum spraying conditions (for example, spraying particle size, spraying temperature, a spraying volume, spraying time, etc.) depending on characteristics of the chemicals to be sprayed (for example, types, viscosity, evaporation rates, diffusion properties, penetrating properties, toxicity, safety, spraying periods, spraying temperature, a spraying amount for one time, spraying particle size, residual periods of chemicals, etc.).

It is another object of the present invention to provide an ultra-low volume spraying device capable of preventing blockage of a spray nozzle by compressed gas used to spray chemicals.

It is a further object of the present invention to provide an ultra-low volume spraying device capable of reading characteristic information of chemicals to be sprayed, which is recorded in one side of a chemical container, and allowing actual spraying information (for example, sprayed conditions and sprayed time and date) of chemicals to be stored in a memory or be transferred to the outside through wire-wireless communication, thereby enabling the information to be utilized in an agricultural product traceability system, a farming daily record, or the like.

### [Technical Solution]

In accordance with an aspect of the present invention, the above and other objects can be accomplished by the provision of an ultra-low volume spraying device including a chemical container to receive chemicals therein, a gas supply part to supply compressed gas, a nozzle part to eject the chemicals mixed with the compressed gas, a connection member including a first path to guide the compressed gas and a second path to guide the chemicals to the nozzle part while connecting the chemical container to the nozzle part, and a cover part to selectively open and close an exit of at least one of the first and second paths, wherein the second path is able to adjust an opening degree of the exit corresponding to characteristic information which exhibits characteristics of the chemicals.

The connection member may include a connection part installed at a closure part of the chemical container and a connection pipe connected to the connection part so as to provide the first and second paths.

The closure part may include therein a protrusion piece capable of adjusting a height thereof depending on characteristics of the chemicals and a fixing piece to fix a position of the protrusion piece.

The connection pipe may include a liquid pipe communicating with the chemical container and serving as the second path accommodated within the first path, an ejection control rod to adjust a sectional area of a liquid pipe ejection hole formed at an exit of the liquid pipe corresponding to the height of the protrusion piece, and an elastic member to restrict movement of the ejection control rod.

The nozzle part may be provided with a compressed gas ejection hole formed between the exit of the first path and the liquid pipe, and the nozzle part may be provided, at one end thereof, with a nozzle protrusion mounted to an exit side of the compressed gas ejection hole and liquid pipe ejection hole.

The cover part may include a cover capable of opening and sealing the nozzle part by pivoting thereof and a cover driver to pivot the cover, and when the cover seals the nozzle part, the cover may come into contact with the nozzle protrusion and the compressed gas ejected to the compress gas ejection hole may be introduced into the liquid pipe ejection hole.

The closure part may be mounted with a coupling protrusion, it being determined whether or not the coupling protrusion protrudes depending on characteristics of the chemicals received in the chemical container, and the connection part may include a switch to detect mounting of the coupling protrusion.

The closure part may have an upper surface mounted with a magnetic substance, it being determined whether or not the magnetic substance exhibits magnetism depending on characteristics of the chemicals received in the chemical container, and the connection part may include a magnetic switch which is mounted to a contact surface coming into contact with the upper surface so as to detect mounting of the magnetic substance.

The closure part may include a data storage part to store the characteristic information of the chemicals received in the chemical container, and the connection part may include a data reader to read the characteristic information of the chemicals stored in the data storage part.

The ultra-low volume spraying device may further include a microcomputer to store the characteristic information of the chemicals provided from any one of the switch, magnetic switch, and data reader and sprayed information relative to actual spraying of the chemicals in a memory, and a communication part to transmit the information stored in the memory by wire or wireless communication.

The gas supply part may include a heater to apply heat and a compressed gas valve to adjust supply of the compressed gas, and the compressed gas may be at least one of carbon dioxide and air.

The ultra-low volume spraying device may further include an air transfer pipe to accommodate the nozzle part and a blower to move the chemicals ejected from the nozzle part.

The air transfer pipe may be formed with air exits which are spaced apart from one another by a predetermined distance, and the air exits may be mounted with electrostatic generating electrodes, respectively.

The chemical container may be coupled to or separated from the connection member.

In accordance with another aspect of the present invention, there is provided an ultra-low volume spraying device including a chemical container to receive chemicals therein, a gas supply part to supply compressed gas, a nozzle part to eject the chemicals mixed with the compressed gas, a connection member including a first path to guide the compressed gas and a second path to guide the chemicals to the nozzle part while connecting the chemical container to the nozzle part, a data storage part to store kinds of the chemicals received in the chemical container and information relative to spraying characteristics, and a data reader to read the information of the data storage part, wherein the second path is able to adjust an opening degree of an exit thereof corresponding to the read information.

The data storage part may encode and record the information, wherein the data storage part may be a bar code type or a radio frequency identification (RFID) chip type, and the data reader may be a bar code reader or a RFID reader.

The ultra-low volume spraying device may further include a heater installed at the connection pipe so as to heat the chemicals guided by the second path.

The ultra-low volume spraying device may further include a valve seat installed within the connection pipe and extending from an exit of the second path, and a valve seat adjuster to adjust a position of the valve seat depending on the read information.

The ultra-low volume spraying device may further include an eddy air generator installed at an outer side of the exit of the second path so as to generate eddy air using the compressed gas supplied through the first path, and a venturi tube to guide the eddy air and to form negative pressure, wherein the eddy air generator may include a plurality of eddy air generators separately installed at front and rear sides of the venturi tube.

The data storage part may be mounted to a closure part of the chemical container, and the data reader may be mounted to the connection member.

The ultra-low volume spraying device may further include a microcomputer to control the heater based on the read information provided from the data reader.

The ultra-low volume spraying device may further include a microcomputer to control the valve seat adjuster based on the read information provided from the data reader.

The ultra-low volume spraying device may further include a microcomputer to store the information of the chemicals, which are received in the chemical container, provided from the data reader and sprayed time and date relative to spraying of the chemicals in a memory, and a communication part to transmit the information stored in the memory by wire or wireless communication.

The communication part may include a universal serial bus (USB) interface which transmits information stored in the memory to a USB memory electrically connected to the microcomputer.

The information stored in the memory may be encoded so as to be decoded by a particular program, thereby being utilized as base data for an agricultural product traceability system or a farming daily record.

### [Advantageous Effects]

In accordance with an ultra-low volume spraying device according to the present invention, since liquid chemicals are sprayed in the form of ultrafine particles without dilution with water, it may be possible to solve problems of the above-mentioned atomizing type spraying device. Also, since the chemicals are automatically sprayed using characteristic information of the chemicals, which is encoded and recorded in a chemical container, the chemicals may be sprayed under optimum spraying conditions taking into consideration characteristics of each chemical even if a user does not take additional actions. Thus, the present invention has an effect of enabling optimization of spraying efficiency and pest control effects.

In addition, in accordance with an ultra-low volume spraying device according to the present invention, compressed gas used to spray chemicals flows back into a path from which the chemicals are ejected, thereby being easily able to prevent blockage of a spray nozzle. Also, it may be possible to achieve activation of microorganism agents by heating and agitation of the chemicals within a container.

In addition, in accordance with an ultra-low volume spraying device according to the present invention, spraying particles accumulate electrostatic charge during spraying of chemicals and have kinetic energy by a blower, thereby being able to maximize adhesive efficiency of the spraying particles.

Furthermore, in accordance with an ultra-low volume spraying device according to the present invention, characteristic information, which is recorded in a chemical container, is automatically read and stored in the ultra-low volume spraying device during mounting of the chemical container, thereby being able to provide the information as objective base data for an agricultural product traceability system, a farming daily record, or the like, together with actual spraying information (for example, sprayed conditions and sprayed time and date).

### [Description of Drawings]

The above and other objects, features and other advantages of the present invention will be more clearly understood from the following detailed description taken in conjunction with the accompanying drawings, in which:
FIG. 1 is a conceptual view illustrating an ultra-low volume spraying device according to a first embodiment of the present invention;
FIG. 2 is a view illustrating a state in which a cover shown in FIG. 1 seals a nozzle part;
FIG. 3 is a view illustrating a cover different from FIG. 1 according to another embodiment;
FIG. 4 is a view illustrating a state in which an ejection control rod is moved by a protrusion piece;
FIG. 5A is a view illustrating an upper surface of a closure part;
FIG. 5B is a conceptual view illustrating a contact state between coupling protrusions and switches at the upper surface of FIG. 5A in a spread form;
FIG. 6A is a view illustrating a closure part provided with magnetic substances according to another embodiment;
FIG. 6B is a view illustrating a contact state between magnetic substances and magnetic switches at an upper surface of FIG. 6A;
FIG. 7 is a view illustrating a state in which chemicals are ejected through an air transfer pipe;
FIG. 8 is a view illustrating a state in which chemicals are ejected through a plurality of air transfer pipes;
FIG. 9 is a conceptual view illustrating an ultra-low volume spraying device according to a second embodiment of the present invention;
FIG. 10 is an enlarged view illustrating portion 'A' shown in FIG. 9;
FIG. 11 is an exploded view illustrating an vortical air type nozzle to form spraying chemicals in the form of fine particles;
FIG. 12A is a view illustration an upper surface of a closure part;
FIG. 12B is a view illustrating a state in which container side data are recognized by a spraying device side data reader during coupling between a chemical container and the spraying device in FIG. 12A; and
FIG. 13 is a conceptual view illustrating data flow and an electronic control unit of the spraying device.

### [Best Mode]

Hereinafter, exemplary embodiments of the present invention will be described in more detail with reference to the accompanying drawings so as to be easily realized by those skilled in the art. The present invention may, however, be embodied in different forms and should not be constructed as limited to the embodiments set forth herein.

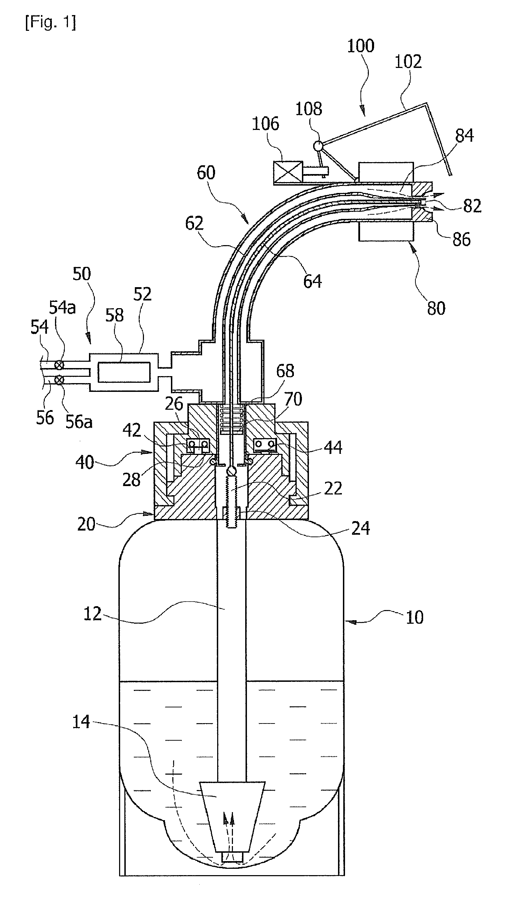

FIG. 1 is a conceptual view illustrating an ultra-low volume spraying device according to a first embodiment of the present invention. The following description will be given below with reference to FIG. 1.

The ultra-low volume spraying device according to the first embodiment of the present invention includes a chemical container 10 receiving chemicals (for example, liquid chemicals) therein while being provided, at an inlet thereof, with a closure part 20, a connection part 40 mounted to the closure part 20 of the chemical container 10, a connection pipe 60 extending from the connection part 40, a gas supply part 50 mounted to one side of the connection pipe 60 so as to supply compressed gas to the connection pipe 60, a nozzle part 80 formed at one end of the connection pipe 60 so as to eject the chemicals mixed with the compressed gas, and a cover part 100 to adjust opening and closing of the nozzle part 80.

The chemical container 10 receives chemicals to be sprayed onto crops, and the chemicals may be sold ready for use, eliminating the need for operations such as dilution with water.

Meanwhile, the chemical container 10 is provided therein with a pipe 12 extending from the closure part 20, and the pipe 12 is mounted, at an end thereof, with a filter 14 to filter out foreign matter in the chemicals introduced through the pipe 12.

The closure part 20 is provided therein with a protrusion piece 22 capable of adjusting a height and a fixing piece 24 to fix a position of the protrusion piece 22. The fixing piece 24 may be fixed to an inner surface of the closure part 20 while being formed, at an inner side thereof, with a thread. The protrusion piece 22 may be formed, at an outer side thereof, with a thread engaged with the thread formed at the inner side of the fixing piece 24. Particularly, the protrusion piece 22 may have a shape similar to a bolt and be fixed in a state in which a position thereof is moved within the fixing piece 24.

The connection part 40 is installed between the closure part 20 and the connection pipe 60. The connection pipe 60 is fixed to the closure part 20 so as to move the chemicals through the connection pipe 60.

The connection pipe 60 includes a liquid pipe 62 accommodated therein to move the chemicals, an ejection control rod 64 accommodated within the liquid pipe 62, and a spring 68 (or another elastic member capable of performing the same function) to restrict movement of the ejection control rod 64.

In this case, the ejection control rod 64 is pressed by the protrusion piece 22, thereby enabling an opened sectional area of a liquid pipe ejection hole 82 to be adjusted.

The spring 68 is mounted, at one end thereof, with a spring fixing piece 70, and the spring fixing piece 70 is moved integrally with the ejection control rod 64. For example, when the ejection control rod 64 is, at an end thereof, pushed by the protrusion piece 22, the spring 68 is compressed and thus the ejection control rod 64 is lifted.

On the other hand, when the protrusion piece 22 is located down, the spring 68 is returned to an original state while moving the spring fixing piece 70 in a downward direction, and thus the ejection control rod 64 is moved along the spring fixing piece 70.

The nozzle part 80 is provided with a compressed gas ejection hole 84 formed between the connection pipe 60 and the liquid pipe 62. The nozzle part 80 is formed, at one end thereof, with a nozzle protrusion 86 mounted to the outside than the compressed gas ejection hole 84 and liquid pipe ejection hole 82.

The chemicals are moved through the liquid pipe ejection hole 82, whereas the compressed gas, namely, compressed air or carbon dioxide gas is emitted through the compressed gas ejection hole 84. Meanwhile, the nozzle protrusion 86 may be installed to partially block a sectional area of the compressed gas ejection hole 84.

The cover part 100 includes a cover 102 capable of sealing the nozzle part 80 and a solenoid 106 which is a cover driver to pivot the cover 102. The cover 102 may have a variety of shapes. For example, the cover 102 may have any shape which is able to selectively seal the nozzle part 80.

The cover part 100 is provided with a fixing shaft 108 to pivot the cover 102, and the cover 102 may be pivoted or slide about the fixing shaft 108.

When the cover 102 is pivoted by the solenoid 106, an inner surface of the cover 102 comes into contact with the nozzle protrusion 86, to seal the nozzle part 80.

The gas supply part 50 includes a tube 52 communicating with the connection pipe 60, a carbon dioxide supply tube 54 communicating with the tube 52, and a compressed air supply tube 56 communicating with the tube 52. The carbon dioxide supply tube 54 may supply carbon dioxide gas, whereas the compressed air supply tube 56 may supply compressed air.

In particular, the tube 52 may be mounted with a heater 58 to supply heat, the carbon dioxide supply tube 54 may be mounted with a carbon dioxide valve 54a to adjust a movement amount of carbon dioxide, and the compressed air supply tube 56 may be mounted with a compressed air valve 56a to adjust supply of compressed air. Thus, in accordance with such a configuration, the carbon dioxide and compressed air may be selectively supplied.

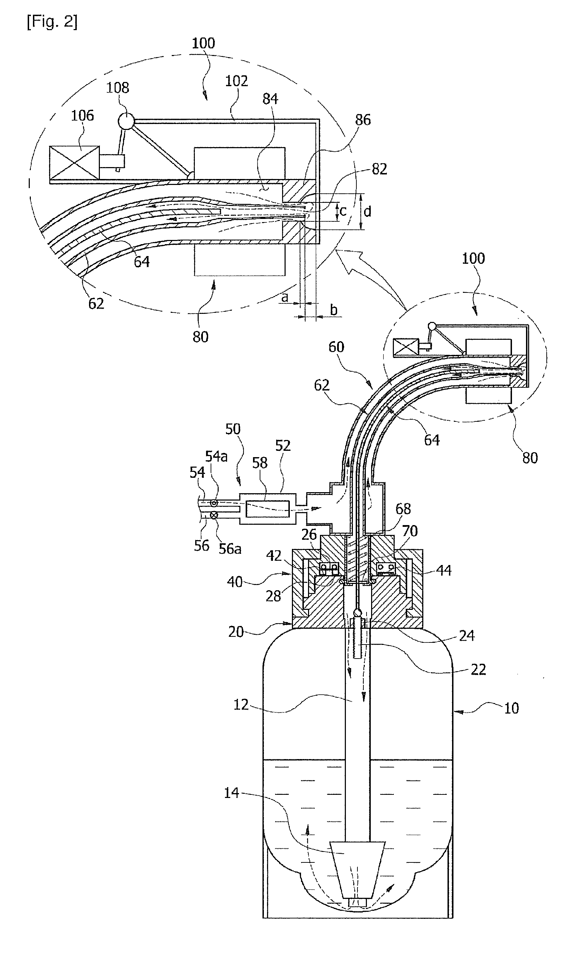

FIG. 2 is a view illustrating a state in which the cover shown in FIG. 1 seals the nozzle part. FIG. 2 shows that the ultra-low volume spraying device performs cleaning and agitation functions. The following description will be given below with reference to FIG. 2.

When power is applied to the solenoid 106 and it is operated, the cover 102 connected to one end of the solenoid 106 is pivoted. The inner surface of the cover 102 comes into contact with the nozzle protrusion 86, to seal the nozzle part 80.

Although FIG. 2 shows that the carbon dioxide valve 54a is opened so that carbon dioxide is supplied to the liquid pipe 62, the compressed air valve 56a may be opened so that compressed air is supplied to the liquid pipe 62.

The following description will be given on the basis of carbon dioxide gas. When the carbon dioxide gas valve 54a is opened, the carbon dioxide gas passes through the tube 52 and is introduced into the outside of the liquid pipe 62. The carbon dioxide gas passes through the compressed gas ejection hole 84 and strikes the inner surface of the cover 102, thereby preventing the carbon dioxide gas from being emitted to the outside of the nozzle part 80.

The outside of the liquid pipe 62 has high pressure due to the carbon dioxide gas, whereas the inside of the liquid pipe 62 has low pressure. Thus certain amount of carbon dioxide gas is introduced into the chemical container 10 through the inside of the liquid pipe 62.

In this case, one side of the chemical container 10 (for example, one side of the closure part 20) is formed with an outlet (not shown) so that the carbon dioxide introduced into the chemical container 10 may be discharged to the outside of the chemical container 10. Accordingly, it is preferable to achieve continuous cleaning and agitation.

Also, the outlet is constituted so as to be opened to the outside of the chemical container 10 manually. Thus, it is preferable to open the outlet when the chemicals are normally ejected (that is, negative pressure is generated within the chemical container).

Chemicals having high viscosity pass within the liquid pipe ejection hole 82 and the liquid pipe 62. In this case, since the chemicals are ejected by Venturi effect due to high pressure gas discharged through the compressed gas ejection hole 84, a blockage phenomenon may be generated. However, since high pressure carbon dioxide gas is supplied within liquid pipe ejection hole 82 and the liquid pipe 62 by the above-mentioned action, it can be cleaned and prevent blockage of the liquid pipe ejection hole 82, liquid pipe 62, and filter 14.

A distance between the nozzle protrusion 86, the compressed gas ejection hole 84, and liquid pipe ejection hole 82 may be set up as shown in FIG. 2 so that the carbon dioxide gas may be smoothly introduced from the outside of the liquid pipe 62 to the inside of the liquid pipe 62. That is, the liquid pipe ejection hole 82 extends from one end of a groove formed at the nozzle protrusion 86 by a predetermined distance 'a' while being located inward from an end of the nozzle protrusion 86 by a predetermined distance 'b'.

Also, it is preferable that the compressed gas ejection hole 84 has a smaller diameter 'c' than a maximum diameter 'd' of the groove formed at nozzle protrusion 86.

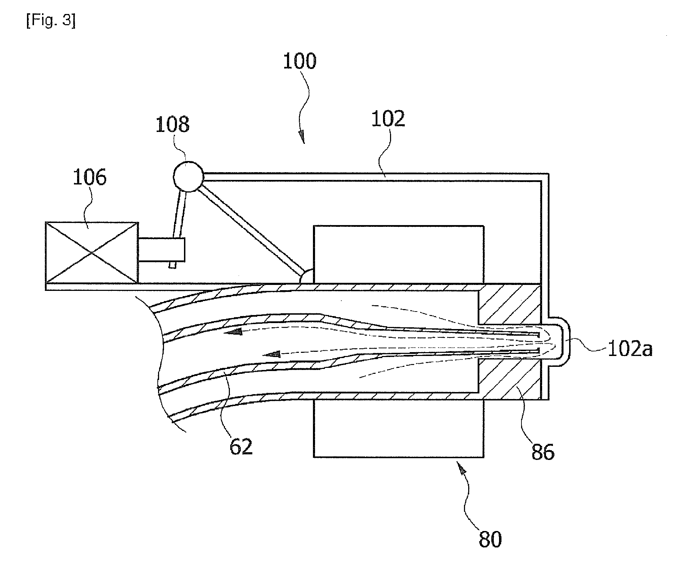

FIG. 3 is a view illustrating a cover according to another embodiment. The following description will be given below with reference to FIG. 3. In FIG. 3 different from FIG. 2, a bent piece 102a is formed at a cover 102. The bent piece 102a protrudes outward so that the bent piece 102a is provided, at an inner side thereof, with a space of a predetermined size. Thus, the carbon dioxide gas may flow back from the outside of the liquid pipe 62 to the inside thereof through the space.

When the bent piece 102a is formed, there is no limitations as to positions of the liquid pipe ejection hole 82, compressed gas ejection hole 84, and nozzle protrusion 86 as shown in FIG. 2.

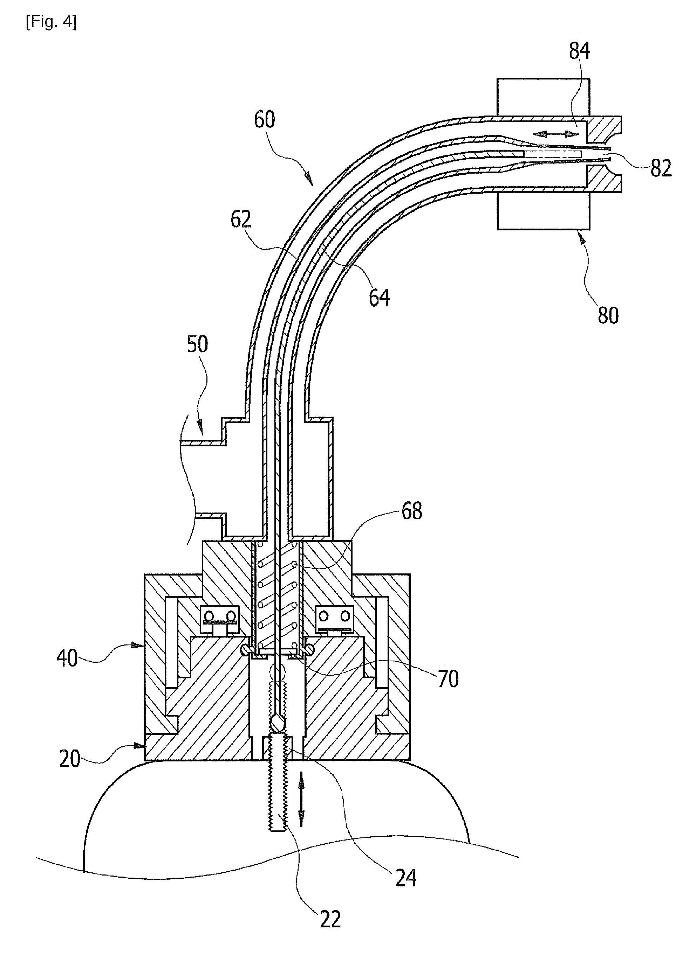

FIG. 4 is a view illustrating a state in which the ejection control rod is moved by the protrusion piece. The following description will be given below with reference to FIG. 4.

Although the protrusion piece 22 is fixed to the fixing piece 24, the protrusion piece 22 may be located at varied heights depending on various kinds or characteristics of chemicals received in the chemical container 10.

For example, when the protrusion piece 22 is located down, the ejection control rod 64 is located down, and the ejection control rod 64 is moved, at one end thereof, inward of the liquid pipe ejection hole 82, thereby enabling the opened sectional area of the liquid pipe ejection hole 82 to be increased. In this case, when compressed air is ejected to the compressed gas ejection hole 84, a large amount of chemicals surely be ejected.

On the other hand, when the protrusion piece 22 is located up, the ejection control rod 64 is located up, and the ejection control rod 64 is moved, at one end thereof, rearward of the liquid pipe ejection hole 82, thereby enabling the opened sectional area of the liquid pipe ejection hole 82 to be decreased. In this case, even when the compressed air having the same pressure is ejected to the compressed gas ejection hole 84, a small amount of chemicals may be ejected.

This is because the liquid pipe ejection hole 82 has a shape in which the sectional area thereof is gradually decreased, namely, a cone shape in proportion to approach from one end thereof to the other end thereof. The opened sectional area of the liquid pipe ejection hole 82 to eject chemicals may be varied according to any position of the end of the ejection control rod 64 having the same thickness within the liquid pipe ejection hole 82.

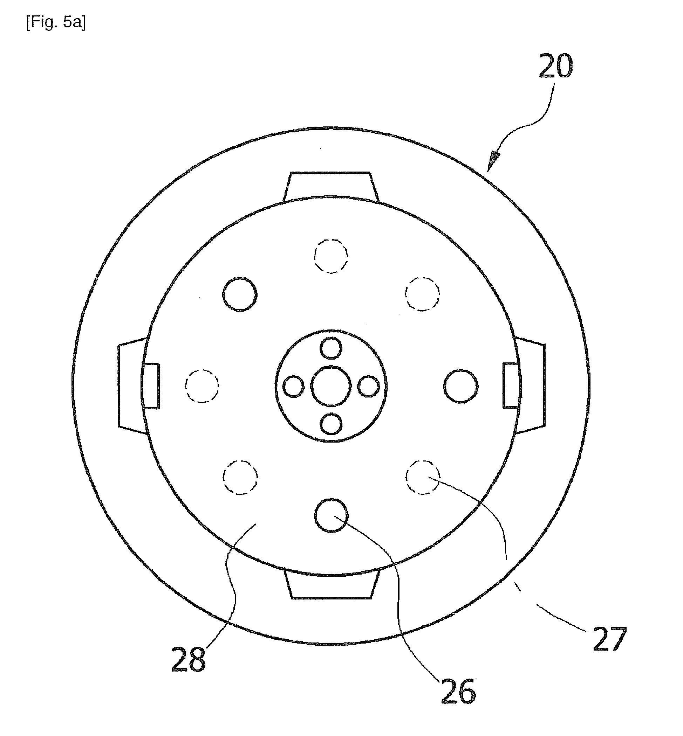

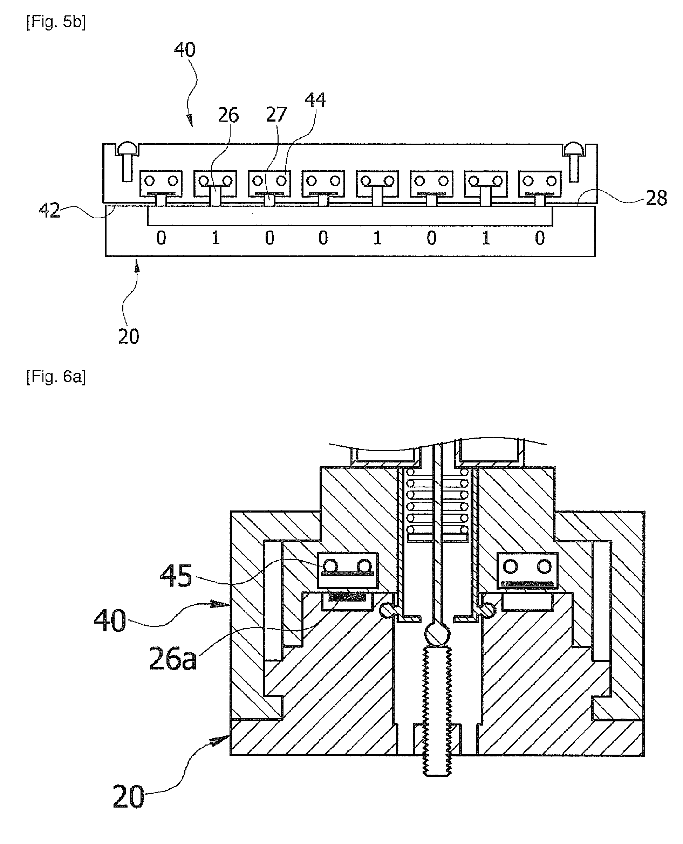

FIG. 5A is a view illustrating an upper surface of the closure part. FIG. 5B is a conceptual view illustrating a contact state between coupling protrusions and switches at the upper surface of FIGS. 1 and 5A in a spread form. The following description will be given below with reference to FIGS. 1, 5A and 5B.

The closure part 20 has an upper surface 28 provided with the coupling protrusions 26, wherein it is determined whether or not the coupling protrusions 26 protrude depending on characteristics of chemicals received in the chemical container 10. Portions to which the coupling protrusions 26 are mounted and portions 27 to which the coupling protrusions 26 are not mounted may exist at mounting positions of the coupling protrusions 26. Mounting of the coupling protrusions 26 at the corresponding positions may be varied depending on characteristics of chemicals.

The connection part 40 includes the switches 44 which are mounted to a contact surface 42 coming into contact with the upper surface 28 so as to detect the mounting of the coupling protrusions 26. As shown in FIG. 5B, since the switches 44 are pressed upward by protrusion shapes of the coupling protrusions 26, the switches 44 may recognize characteristic information of chemicals received in the chemical container 10 (for example, name, types, viscosity, evaporation rates, diffusion properties, penetrating properties, toxicity, safety, spraying periods, spraying temperature, a spraying amount for one time, spraying particle size, residual periods of chemicals, etc. (hereinafter, referred to as 'characteristic information')).

Particularly, a plurality of coupling protrusions 26 and switches 44 may be provided to recognize various characteristics of chemicals.

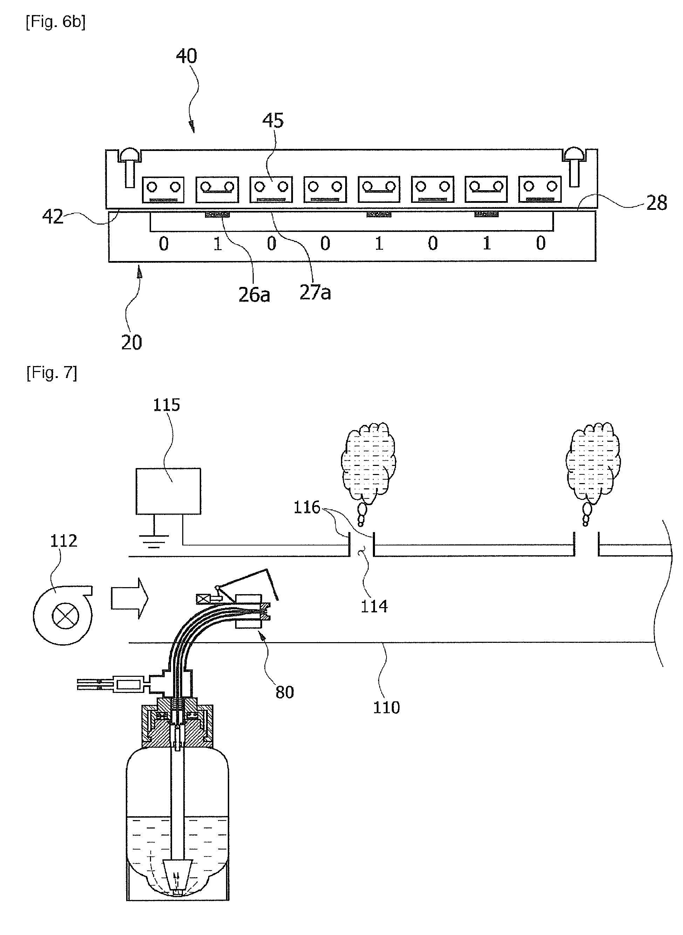

FIG. 6A is a view illustrating a closure part provided with magnetic substances according to another embodiment. FIG. 6B is a view illustrating a contact state between magnetic substances and magnetic switches at an upper surface of FIG. 6A. The following description will be given below with reference to FIGS. 6A and 6B.

The closure part 20 has the upper surface 28 provided with the magnetic substances 26a, wherein it is determined whether or not the magnetic substances 26a exhibit magnetism depending on characteristics of chemicals received in the chemical container 10. Unlike the coupling protrusions shown in FIGS. 5A and 5B, the characteristic information of chemicals may be recognized using the magnetic substances 26a.

Similarly, the connection part 40 includes the magnetic switches 45 which are mounted to the contact surface 42 coming into contact with the upper surface 28 so as to detect the mounting of the magnetic substances 26a. The characteristic information of chemicals may be recognized using the magnetic substances 26a similarly to the coupling protrusions 26, but there is no need for the magnetic substances 26a to come into contact with the magnetic switches 45.

In another example, the above-mentioned characteristic information of chemicals may be recorded in a data storage part (not shown) such as a bar code or a radio frequency identification (RFID) chip to be described in a second embodiment later, and be mounted to the closure part 20. In this case, the connection part 40 is provided with a data reader (not shown) such as a bar code reader or a RFID reader so as to recognize the characteristic information of chemicals.

In order for the data stored in the data storage part to be not arbitrarily modified by a user, the data may be encoded so as to be decoded only by a particular program.

FIG. 7 is a view illustrating a state in which chemicals are ejected through an air transfer pipe. The following description will be given below with reference to FIG. 7.

The ultra-low spraying device according to the present embodiment may further include an air transfer pipe 110 to accommodate the nozzle part 80 and a blower 112 mounted to the air transfer pipe 110 so as to move chemicals ejected from the nozzle part 80.

The chemicals ejected through the nozzle part 80, namely, the liquid pipe ejection hole 82 may be widely spread by air having constant pressure generated from the blower 112.

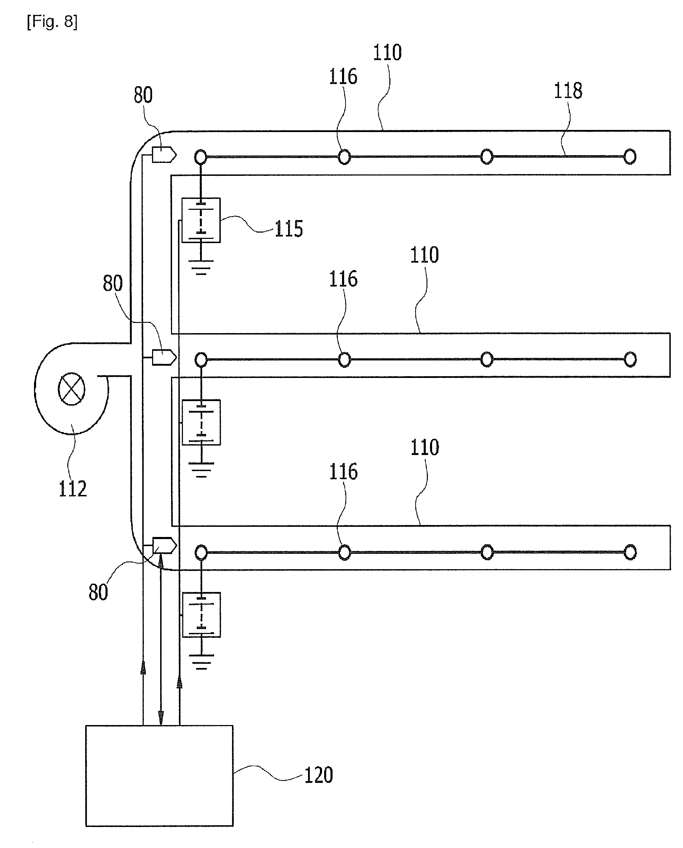

The air transfer pipe 110 is formed with air exits 114 which are spaced apart from one another by a predetermined distance, and the air exits 114 may be respectively mounted with electrostatic generating electrodes 116. High voltage of direct current is generated by high voltage generator 115 may be supplied to the electrostatic generating electrodes 116 through a high voltage shield cable 118 (shown in FIG. 8, instead of FIG. 7).

The chemicals may be ejected to the outside of the air transfer pipe 110 through the air exits 114. In this case, the droplets emitted from a nozzle part 80 is positively charged while passing a vicinity of the electrostatic generating electrode 116, whereas earth potential has a negative charge induced on its surface by attraction of electrons, thereby easily adhering to plants having inverse charge.

Also, an operation of the ultra-low spraying device according to the present embodiment is controlled by a control unit 120 (shown in FIG. 8, instead of FIG. 7) such as a microcomputer. The control unit 120 controls operations of the heater 58, gas supply part 50, solenoid 106, blower 112, and high voltage generator 115 using the characteristic information of chemicals recognized through the above-mentioned switches 44, magnetic switches 45, bar code reader, or RFID reader.

FIG. 8 is a view illustrating a state in which chemicals are ejected through a plurality of air transfer pipes. The following description will be given below with reference to FIG. 8.

A plurality of air transfer pipes 110 is installed, and each nozzle part 80 is mounted within a beginning portion of a linear portion of the associated air transfer pipe 110. One blower 112 is connected to one end of each of the plural air transfer pipes 110, thereby achieving reduction in manufacturing costs. The air supplied by the blower 112 may allow the chemicals ejected through each nozzle part 80 to be widely spread, thereby enabling shortening of pest control time.

The control unit 120 is connected to the nozzle parts 80 so as to detect the characteristic information of chemicals ejected through the nozzle parts 80, thereby controlling actual spraying conditions of the chemicals ejected through the nozzle parts 80.

In this case, the control unit 120 detects the characteristic information of chemicals using information read from the coupling protrusions, magnetic substances, bar code, or RFID chip of the closure part 20 through the above-mentioned switches 44, magnetic switches 45, bar code reader, or RFID reader of the connection part 40.

The above-mentioned ultra-low volume spraying device operates as follows. The following description will be given below with reference to FIGS. 1, 2 and 3.

Compressed air having constant pressure and temperature is introduced into the connection pipe 60 through the gas supply part 50, heats liquid flowing within the liquid pipe 62 by heat exchange while passing through the connection pipe 60, namely, an outer peripheral portion of the liquid pipe 62, and is ejected to the compressed gas ejection hole 84.

Vacuum pressure (or negative pressure) is formed in the vicinity of the liquid pipe ejection hole 82 by pressure drop rapidly generated in the vicinity of the compressed gas ejection hole 84, and thus chemicals is ejected from the liquid pipe 62 by the vacuum pressure. In this case, the liquid is formed in the form of fine particles by a large amount of compressed gas and is normally sprayed.

Meanwhile, in the case of performing nozzle cleaning and agitation functions, power is applied to the solenoid 106, and the solenoid 106 pivots the cover 102 about the fixing shaft 108. Consequently, the cover 102 closes the nozzle part 80.

When nozzle cleaning, chemical heating, and chemical agitation are regularly or arbitrarily performed by a processing command input to the control unit 120, the cover 102 closes the nozzle part 80 and a certain amount of compressed gas flows back into the liquid pipe 62 so as to be introduced into the chemical container 10, thereby enabling removal of foreign matter fixed within the liquid pipe 62. Also, the chemical in the chemical container 10 are agitated and diluted so as to maintain the chemical at proper temperature.

Unlike the related art, the present invention may particularly perform a cleaning mode by forming the liquid pipe ejection hole 82, the compressed gas ejection hole 84, and the nozzle protrusion 86 in a shape as shown in FIG. 2.

FIG. 3 shows another example of performing a cleaning function of the liquid pipe and an agitation function of chemicals, wherein compressed gas ejected through the compressed gas ejection hole 84 as shown in FIGS. 1 and 2 may flow back into the liquid pipe 62 by the bent piece 102a.

In the conventional ultra-low volume spraying device, since spraying particles are very small, it may be impossible that chemicals are adjusted in particle size suitable for the chemicals and sprayed. However, in accordance with the ultra-low volume spraying device of the present invention, chemicals may be sprayed according to various characteristics of spraying chemicals by configuration of connecting the chemical container to the connection part.

The following description will be given below with reference to FIGS. 1 and 4. In the chemical container 10, the height of the protrusion piece 22 is adjusted depending on a set value calculated taking into consideration intrinsic characteristic of each chemical. As shown in FIG. 4, the position of the ejection control rod 64 may be varied depending on chemicals, and movement of the ejection control rod 64 may be limited by the spring 68.

That is, the opened sectional area of the liquid pipe ejection hole 82 is gradually decreased as one end of the ejection control rod 64 gets closer to the end of the liquid pipe ejection hole 82. Therefore, even when compressed air having the same pressure is supplied, a sprayed amount of chemicals is decreased.

In this case, the ejection control rod 64 is moved in an exit direction of the liquid pipe ejection hole 82 so as to restrict chemical flow in the liquid pipe ejection hole 82. Consequently, a flow rate of liquid is decreased and a sprayed amount of chemicals is reduced.

On the other hand, the opened sectional area of the liquid pipe ejection hole 82 is gradually increased as one end of the ejection control rod 64 gets closer to the inside of the liquid pipe ejection hole 82. Therefore, when compressed air having the same pressure is supplied, a sprayed amount of chemicals is increased.

The position of the ejection control rod 64 may be adjusted by the height of the protrusion piece 22, and the height of the protrusion piece 22 may be varied depending on characteristics of chemicals received in the chemical container 10 by a producer.

In this case, the height of the protrusion piece 22 functions as the characteristic information of chemicals received in the chemical container 10.

In order to obtain ideal spraying effects in the ultra-low volume spraying device, spraying conditions must be maintained on a per chemical basis according to various chemical characteristics. Particularly, in the case of microorganism agents, it is preferable to optimally maintain spraying conditions also before spraying of the chemicals. For example, it is preferable that when the spraying chemicals have high viscosity or ambient temperature is low, the spraying chemicals must be heated to be properly sprayed.

In this case, carbon dioxide gas or compressed air may be heated using the heater 58 mounted to the tube 52. Particularly, in order to form optimum spraying conditions in microorganism agents, it is preferable that spraying chemicals are maintained at constant temperature depending on the characteristic information of the chemicals received in the chemical container. Also, it is preferable that the spraying chemicals are activated by supply of properly heated air for a predetermined time and is then sprayed.

The following description will be given below with reference to FIGS. 5A and 5B. The coupling protrusions 26 are mounted to the upper surface 28 of the closure part 20, and the connection part 40 includes the switches 44 which are mounted to the contact surface 42 coming into contact with the upper surface 28. The information detected by the coupling protrusions 26 and the switches 44 includes information relative to types and spraying characteristics of chemicals received in the chemical container 10.

That is, an on/off value is obtained according to whether or not the switch 44 are respectively pressed by the coupling protrusions 26, and thus the information relative to the chemicals may be recognized.

As shown in FIG. 5A, among the eight portions of the upper surface 28, the coupling protrusions 26 may be mounted to only the three portions, whereas the other portions may be maintained as the portions 27 to which the coupling protrusions 26 are not mounted. As shown in FIG. 5B, coupling relations may be maintained between the respective switches 44 and the respective coupling protrusions 26. In FIG. 5B, '1' signals may be respectively obtained in the portions to which the coupling protrusions 26 are mounted, whereas '0' signals may be respectively obtained in the portions to which the coupling protrusions 26 are not mounted.

When eight switch modules having an on/off function are provided, 256 data information (for example, characteristic information of chemicals, which is set up and/or encoded by a producer) may be set up by 8-bit input. Similarly, when nine switch modules are provided, 512 data information may be set up, and when ten switch modules are provided, 1024 data information may be set up. Therefore, it may be possible to distinguish agrochemicals having different characteristics and to control actual spraying conditions (for example, operation status of the heater, operation temperature of the heater, ejection pressure of compressed gas, etc.) suitable for each characteristic. Such control may be preferably performed by allowing the control unit 120 included in the spraying device according to the present embodiment to use information recognized by the switches 44 and the like.

Similarly to a second embodiment to be described later, the control unit 120 may store the recognized information in a memory and the like. In the stored data, information relative to actual spraying of chemicals (hereinafter, referred to as 'spraying information') such as actual spraying conditions (for example, sprayed temperature, sprayed periods, a sprayed amount, etc.) and operation time (namely, sprayed time and date) of the spraying device is further stored in addition to the characteristic information, thereby being able to recognize actual sprayed periods of chemicals, residual status of sprayed agrochemicals, etc. Therefore, the data is provided as objective base data for an agricultural product traceability system or a farming daily record.

In this case, in order for the information (namely, characteristic information and spraying information) stored in the memory to be not arbitrarily modified by a user, the information may be encoded so as to be decoded only by the particular program.

Accordingly, in accordance with the ultra-low volume spraying device of the present embodiment, the chemical container including the characteristic information of chemicals is merely coupled to the spraying device, and thus the control unit may recognize various kinds and characteristic information of spraying chemicals, for example, agrochemicals such as insecticides, germicides, and miticides, agrochemical-based materials such as organic phosphorus-based materials and organic chloride-based materials, natural agrochemicals or microbial agrochemicals such as plant extract, and fertilizers. Consequently, it may be possible to spray chemicals under optimum spraying conditions and to manage histories of sprayed chemicals later.

In this case, the characteristic information of each chemical may be obtained in a manner as represented by Table 1 below.

**[Table 1]**

| | | | | | | | |
|---|---|---|---|---|---|---|---|
| 0 | 1 | 0 | 0 | 1 | 0 | 1 | 0 |

For example, when two switches having 8-bit data capacity is provided, 65,536 data information may be input to the switches. Thus, general information of agrochemicals such as kinds of agrochemicals and agrochemical-based materials may be set up in a predetermined bit, and information of chemicals, such as viscosity and volatility, required for a command of spraying conditions may be included in another predetermined bit.

Particularly, the data relative to the agrochemicals sprayed on crops is recorded and stored so that the data may not be arbitrarily operated or modified by a user. Consequently, the data may be provided as the objective data for the agricultural product traceability system.

The following description will be given below with reference to FIGS. 6A and 6B. In the embodiment illustrated in FIG. 6 different from the embodiment illustrated in FIG. 5, the magnetic substances 26a are provided instead of the coupling protrusions 26, and the magnetic switches 45 are provided instead of the switches 44.

In accordance with the magnetic substances 26a and the magnetic switches 45, non-contact effects may be achieved, thereby enabling prevention of switch module contamination. Meanwhile, the switches according to the present invention are not limited to mechanical type switches and magnetic type switches. For example, the switches may be realized by application of proximity sensors such as high frequency type sensors and capacitance type sensors.

Also, the characteristic information using the above-mentioned various switches may be replaced by application of a bar code or RFID technology, instead of the above-mentioned switches.

The following description will be given below with reference to FIG. 7. The particles sprayed during spraying of ultra-low volume liquid adhere to epidermis of plants or insects while being suspended for a long time and eventually fall by gravity on to a horizontal surface. However, the sprayed particles are ultrafine particles having very small particle size, thereby having small kinetic energy. Accordingly, it is required to improve adhesive efficiency of sprayed particles on the lower surface of leaves.

In a general mist type atomizing method, a plurality of atomizing nozzles is installed at an upper portion of a greenhouse and electrostatic generating electrode is mounted to an exit of each atomizing nozzle so as to spray particles. Such an electrostatic type atomizing method to spray particles, however, has a problem of causing a phenomenon in which the sprayed particles are focused onto leaf tips of plants close to the exit of the atomizing nozzle (Lane and Law 1982, "Transient charge transfer in living plants").

In order to improve the phenomenon in which the sprayed particles are focused onto the leaf tips of plants, the present invention allows the sprayed particles to have kinetic energy using a large amount of air, thereby preventing the focused phenomenon and improving adhesive efficiency.

To this end, in accordance with the ultra-low volume spraying device of the present invention, the air transfer pipes 110 spaced apart from one another by a predetermined distance are installed in the greenhouse, the air exits 114 spaced apart from one another by a predetermined distance are provide in each air transfer pipe 110, and the electrostatic generating electrodes 116 are respectively provided adjacent to the air exits 114. The electrostatic generating electrodes 116 are connected through the high voltage shield cables 118.

When the nozzle parts 80 are respectively installed in the air transfer pipes 110 and spraying begins, spraying particles are mixed with a large amount of air generated by the blower 112, thereby increasing kinetic energy of the spraying particles. Also, the spraying particles are maintained at constant acceleration and ejected while passing through the electrostatic generating electrodes 116. In this case, high voltage (10kv ~ 15kv) is applied to the electrostatic generating electrodes 116, so that the ejected particles have electrostatic charge.

In this case, the electrostatic charged spraying particles, which are mixed with air, are emerged from the electrostatic generating electrodes 116, and easily adhere to the lower surface of leaves where disease and insect pests mainly inhabited by electrostatic effects and constant acceleration.

Here, the lower surface of leaves where the back faces of leaves of plants or the disease and insect pests mainly inhabited has a negative charge induced against the electrostatic generating electrodes 116.

The following description will be given below with reference to FIG. 8. When a plurality of spraying devices and blowers is installed in the greenhouse having a relatively great area, there are problems in that installation costs increase and management of the devices are difficult. To solve such problems, the present invention has a configuration in which one blower 112 is installed and a plurality of air transfer pipes 110 is connected to the exit of the blower 112.

In this case, the control unit 120 may control operations of the blower 112, nozzle parts 80, high voltage generators 115.

Meanwhile, when the plural air transfer pipes 110 and nozzle parts 80 are installed in a great greenhouse, among the plural nozzle parts 80, one nozzle part 80 is designated as a basic nozzle part so that the basic nozzle part may be set up to have bidirectional data transmission and control functions with respect to the control unit 120, whereas the other nozzle parts 80 may be set up to have one direction control function.

As described above, the ultra-low volume spraying device according to the first embodiment of the present invention has an effect as follows. It may be possible to automatically maintain optimum spraying states suitable for characteristics of each chemical depending on characteristic information of chemicals (for example, types, viscosity, evaporation rates, diffusion properties, penetrating properties, toxicity, safety, spraying periods, spraying temperature, a spraying amount for one time, spraying particle size, residual periods of chemicals, etc.) included in the chemical container which is detachably coupled to the spraying device and to spray the chemicals, without the additional need for modification of information relative to spraying conditions by a user.

Also, in accordance with the ultra-low volume spraying device according to the present embodiment allows compressed gas used to spray chemicals to periodically flow back into the path (the above-mentioned liquid pipe) from which the chemicals are ejected before and after the operation of the spraying device or during the operation of the spraying device. Consequently, it may be possible to prevent blockage of the nozzle part and to optimally maintain chemicals also before spraying of the chemicals by agitation of the chemicals received in the chemical container.

In order to improve adhesive efficiency of sprayed particles, the ultra-low volume spraying device according to the present embodiment allows the sprayed particles to have kinetic energy using a large amount of air, thereby preventing chemical injury of plants due to the unequally distributing phenomenon of chemicals sprayed and improving adhesive efficiency, compared with the conventional ultra-low volume spraying device.

Furthermore, in accordance with the ultra-low volume spraying device of the present embodiment, the air transfer pipes are installed in the greenhouse, the air exits spaced apart from one another by a predetermined distance are provide in each air transfer pipe, and the electrostatic generating electrodes are respectively provided at the air exits so that spraying particles have electrostatic charge to easily adhere to the lower surface of leaves of plants. Consequently, the sprayed particles may be effectively applied to disease and insect pests mainly inhabited at the lower surface of leaves.

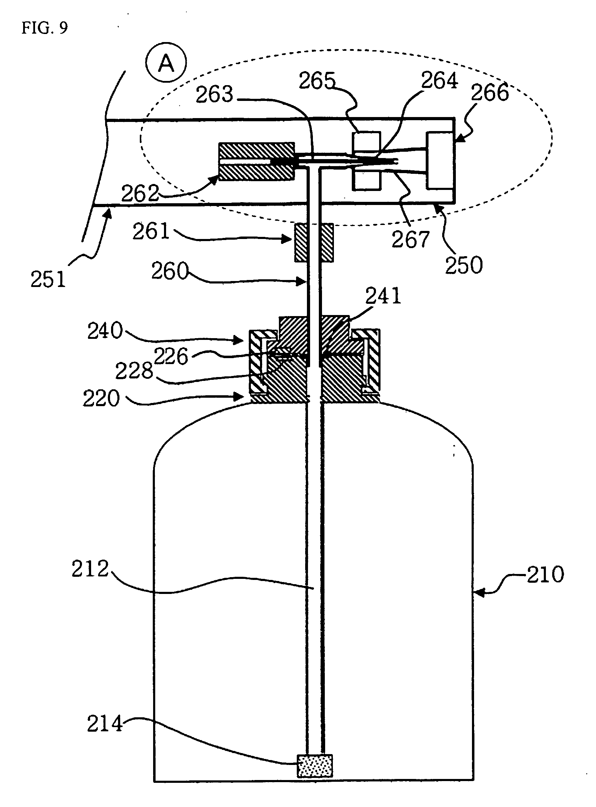

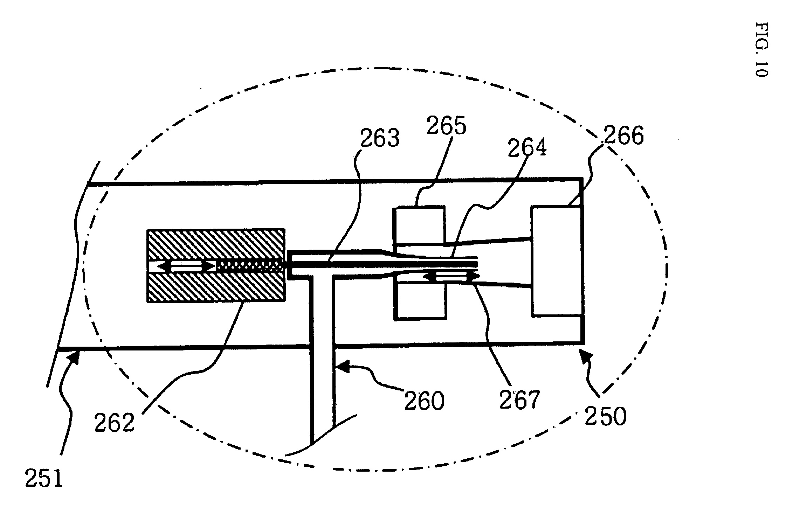

FIG. 9 is a conceptual view illustrating an ultra-low volume spraying device according to a second embodiment of the present invention. FIG. 10 is an enlarged view illustrating portion 'A' shown in FIG. 9. The following description will be given below with reference to FIGS. 9 and 10.

The ultra-low volume spraying device according to the second embodiment of the present invention includes a chemical container 210 receiving chemicals therein while being provided, at an inlet thereof, with a closure part 220, a connection part 240 mounted to the closure part 220 of the chemical container 210, a connection pipe 260 extending from the connection part 240, a liquid ejection port 264 formed at one end of the connection pipe 260 so as to eject the chemicals, a first vortical air generator 265 used to spray the chemicals ejected from the liquid ejection port 264 in the form of small particles, a venturi tube 267 to form vacuum pressure at the liquid ejection port 264, and a second vortical air generator 266 to form the sprayed particles in the form of more smaller particles.

The chemical container 210 receives chemicals to be sprayed, and the chemicals may be sold ready for use, eliminating the need for operations such as measuring chemical and dilution with water.

Meanwhile, the chemical container 210 is provided therein with a liquid pipe 212 extending from the closure part 220, and the liquid pipe 212 is mounted, at an end thereof, with a filter 214 to filter out foreign matter introduced through the liquid pipe 212.

Also, the closure part 220 is provided therein with a liquid path communication with the liquid pipe 212.

The connection part 240 is installed between the closure part 220 and the connection pipe 260. The connection pipe 260 is fixed to the closure part 220 so as to move the chemicals through the connection pipe 260.

The connection pipe 260 is provided, at a predetermined portion thereof, with a heater 261 to heat chemicals.

A packing 241 to maintain air-tightness is mounted to an outer side of the connection pipe 260, which is located at a coupling portion between the connection pipe 260 of the connection part 240 and the liquid path of the closure part 220. The connection pipe 260 is mounted therein with a valve seat 263 extending from an exit of the liquid ejection port 264 so as to control flow of the chemicals ejected through the liquid ejection port 264, and with a valve seat adjuster 262 integrally or separately formed with the valve seat 263 so as to adjust a position of the valve seat 263.

In this case, a position of the valve seat 263 is adjusted by the valve seat adjuster 262 equipped with a position recognition unit operated by a drive unit such as a hydraulic unit, a solenoid, or a step motor. Consequently, a sectional area of the liquid ejection port 264 is changed to adjust flow (or an ejection amount) of chemicals.

The liquid ejection port 264 is provided, at an outer side thereof, with the first vortical air generator 265 to rotate air supplied through a air supply tube 251 at constant pressure so that the chemicals ejected from the liquid ejection port 264 are mixed with the air, the venturi tube 267 to form vacuum pressure (or negative pressure) at an exit of the liquid ejection port 264, and the second vortical air generator 266 to form the sprayed particles in the form of more smaller particles.

In this case, the liquid ejection port 264 has a shape in which the sectional area thereof is gradually decreased, namely, a cone shape in proportion to approach from one end thereof to the other end thereof. The opened sectional area of the liquid ejection port 264 to pass through chemicals is varied according to any position of the end of the valve seat 263 having the same diameter (or thickness) within the liquid ejection port 264.

The position of the valve seat 263 is determined by the characteristic information of chemicals stored in the chemical container 10 as described later. In the case of chemicals having high volatility, the valve seat 263 is moved toward the valve seat adjuster 262 so that the chemicals may be sprayed in the form of large particles taking into consideration volatility. Consequently, the chemical movement path of the liquid ejection port 264 is enlarged, thereby spraying the chemicals in the form of large particles.

On the other hand, in the case of spraying the chemicals in the form of small particles (for example, in the case of the chemicals having low volatility or high toxicity), the valve seat 263 is moved toward the liquid ejection port 264, such that the chemical movement path of the liquid ejection port 264 is narrowed, thereby spraying the chemicals in the form of small particles.

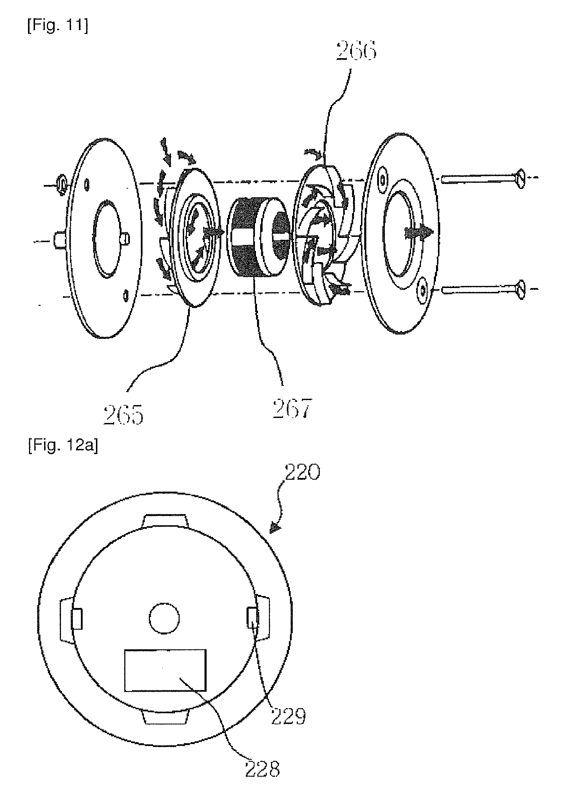

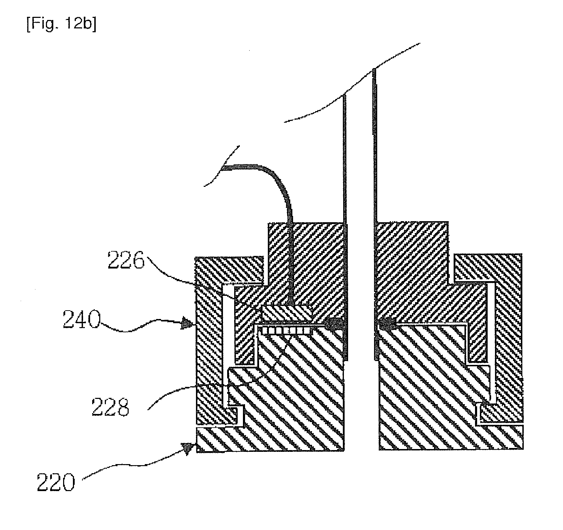

FIG. 12A is a view illustration an upper surface of the closure part 220. FIG. 12B is a view illustrating a state in which a data storage part 228 comes into contact with a data reader 226 in FIGS. 9 and 12A. The following description will be given below with reference to FIGS. 9, 12A, and 12B.

The closure part 220 is provided, at the upper surface thereof, with the data storage part 228 to which characteristic information (for example, name, type, viscosity, evaporation rates, diffusion properties, penetrating properties, toxicity, safety, spraying periods, spraying temperature, a spraying amount for one time, spraying particle size, residual periods of chemicals, etc. as described in the first embodiment) exhibiting characteristics of chemicals received in the chemical container 210 is input.

In this case, the closure part 220 may be provided with position fixing members 229 so that the data storage part 228 exactly corresponds to the data reader 226 to be described later.

The connection part 240 is provided with the data reader 226 which is mounted to a contact surface coming into contact with the data storage part 228 located at the upper surface of the closure part 220.

Although the present embodiment has a configuration in which, for example, the data storage part 228 is comprised of a bar code or a RFID chip, and the data reader 226 is comprised of a bar code reader or a RFID reader, the present invention is not limited thereto. For example, the data storage part 228 and the data reader 226 may be comprised of mechanical type elements (for example, a coupling protrusion or a magnetic substance and a switch or a magnetic switch to recognize the same) as the above-mentioned first embodiment.

Also, in order for the data stored in the data storage part 228 to be not arbitrarily modified by a user, the data may be encoded so as to be decoded only by a particular program.

Although the present embodiment, for example, describes that the data storage part 228 is mounted to the upper surface of the closure part 220, the present invention is not limited thereto. If necessary, the data storage part 228 may be mounted to the inside or outside of the container or any side of the inside and the outside.

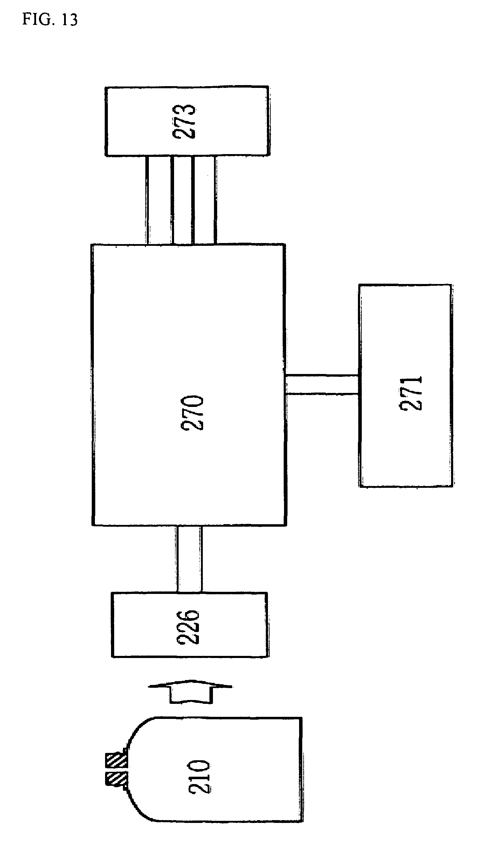

FIG. 13 is a conceptual view illustrating a state in which the information stored in the chemical container 210 is read to be calculated, controlled, and utilized according to a command previously programmed in the memory and the like. The following description will be given below with reference to FIGS. 9 and 13.

When the chemical container 210 including chemicals is coupled to the connection part 240 of the spraying device, the data storage part 228 provided at the closure part 220 of the chemical container 210 while including characteristic information of the chemicals (for example, name, type, viscosity, evaporation rates, diffusion properties, penetrating properties, toxicity, safety, spraying periods, spraying temperature, a spraying amount for one time, spraying particle size, residual periods of chemicals, etc.) is read by the data reader 226 provided at the spraying device side (specifically, the connection part), such that the characteristic information of the chemicals is input to a microcomputer 270.

The information input to the microcomputer 270 is transferred to a central processing unit (CPU) in the microcomputer 270, and the CPU processes (calculates and/or determines) the information in a previously programmed manner and executes a control command.

For example, when the control command is executed to control whether or not power is supplied to the heater 261 provided at the connection pipe 260 and to adjust a spraying amount of chemicals, the microcomputer controls an operation of the heater 261 and simultaneously controls the valve seat adjuster 262 so as to move the valve seat 263 from the liquid ejection port 264 to the valve seat adjuster 262, thereby increasing a spraying amount of chemicals. On the other hand, when the microcomputer controls the valve seat adjuster 262 so as to move the valve seat 263 toward the liquid ejection port 264, a spraying amount of chemicals may be decreased.

Also, data (namely, characteristic information of chemicals) input from the data storage part 228 of the chemical container 210, a control command calculated in the CPU, and information such as sprayed information relative to actual spraying of chemicals, for example, sprayed time and date may be stored in the memory of the microcomputer 270. The stored data may be output through a wire or wireless data transmission unit 271 or be transferred to an outside server. Consequently, the data may be utilized as base data for an agricultural product traceability system or a farming daily record.

In this case, the data transferred or output from the microcomputer 270 using the wire or wireless data transmission unit 271 is processed so as to allow the data to be generally encoded and decoded only by a particular program. Therefore, since a user is not able to process or modify the data, objective sprayed histories may be provided.

Meanwhile, the wire or wireless data transmission unit 271 may include a universal serial bus (USB) interface which transmits information to a USB memory electrically connected to the microcomputer 270.

Also, reference numeral 273 shown in FIG. 13 refers to a driver of the functional devics like heater 261 and valve seat adjuster 262 as described above.

The above-mentioned ultra-low volume spraying device according to the present embodiment operates as follows. The following description will be given below with reference to FIGS. 9 to 13.

Compressed air is supplied to the air supply tube 251 at constant pressure, and passes through the first vortical air generator 265, thereby generating rotational force in a predetermined direction. The rotating air generates a Venturi phenomenon while passing through the venturi tube 267, thereby generating vacuum pressure (or negative pressure) at the exit of the liquid ejection port 264. Subsequently, the air meets with counter rotating air generated by the second vortical air generator 266, thereby being ejected to the outside in the form of small droplet.

In this case, when the chemical container 210 including chemicals is coupled to the connection part 240 of the spraying device, the data storage part 228 provided at the closure part 220 of the chemical container 210 while including characteristic information of the chemicals (for example, name, type, viscosity, evaporation rates, diffusion properties, penetrating properties, toxicity, safety, spraying periods, spraying temperature, a spraying amount for one time, spraying particle size, residual periods of chemicals, etc.) is recognized by the data reader 226 provided at the spraying device side, such that the characteristic information of the chemicals is input to the microcomputer 270.

The information input to the microcomputer is processed at the CPU of the microcomputer 270. For example, information of chemicals to be sprayed is set up as chemicals which have high viscosity and require heating, the CPU executes a control command to heat the chemicals to be sprayed so that power is supplied to the heater 261 provided at the connection pipe 260.

In addition, when the information recorded in the chemical container is information to form spraying particles in the form of large particle size due to spraying chemicals which have high volatility, the CPU executes a command to control the valve seat adjuster 262 so that the valve seat 263 is moved from the liquid ejection port 264 to the valve seat adjuster 262, thereby increasing and maintaining the sectional area of the chemical movement path of the liquid ejection port 264. Consequently, a predetermined amount of spraying chemicals is ejected from the liquid ejection port 264 in the form of relatively large droplet size.

Meanwhile, the chemicals ejected from the liquid ejection port 264 are mixed with air rotated at high speed while passing through the first vortical air generator 265 so as to be finely broken, thereby being formed in the form of fine particles. Subsequently, the spraying chemicals having fine particle size meet with counter rotating air generated by the second vortical air generator 266, thereby generating an vortical phenomenon to form the spraying chemicals in the form of ultrafine particles. Consequently, the chemicals are sprayed in the form of ultrafine particles.

In the conventional ultra-low volume spraying device, since spraying particles are very small, it may be impossible that chemicals are adjusted in particle size suitable for the chemicals and sprayed. However, in accordance with the ultra-low volume spraying device of the present embodiment, information relative to spraying chemicals is input to the closure part of the chemical container 210 including chemicals and is then input to microcomputer of the spraying device before spraying of the chemicals, such that particle size of the chemicals to be sprayed may be automatically adjusted.

Also, in accordance with the ultra-low volume spraying device of the present embodiment, the information input to the data storage part 228 of the chemical container 210 may include information taking into consideration physical and chemical characteristics of spraying chemicals, information relative to agrochemicals such as insecticides, fungicides, miticides, plant extract, and microorganisms, fertilizer, agrochemical-based materials such as organic phosphorus-based materials and organic chloride-based materials, and toxicity, safety, residual periods, and safe health data of agrochemicals, and information such as a arithmetic program of the microcomputer 270 included in the spraying device. The data is input to the microcomputer of the spraying device by merely coupling the chemical container 210 to the spraying device, and thus the microcomputer 270 executes a control command suitable for the input information and stores the input information in the memory.

In this case, in the data stored in the memory, information relative to actual spraying of chemicals such as operation time (namely, sprayed time and date) of the spraying device is further stored in addition to the characteristic information of the chemicals recorded in the chemical container 210, thereby being able to recognize residual status of chemicals sprayed later, etc. Therefore, the data is provided as base data for an agricultural product traceability system or a farming daily record.

In this case, the data may be encoded, stored and decoded only by a particular program so as not to be arbitrarily operated or modified by a user. Therefore, the data may be provided as objective data for an agricultural product traceability system.

As described above, in accordance with the ultra-low volume spraying device according to the second embodiment of the present invention, an opening degree of the liquid ejection port corresponding to the liquid pipe ejection hole of the first embodiment may be controlled using the characteristic information of the chemicals recognized from the chemical container, compared with the above-mention first embodiment. Consequently, it may be possible to automatically and exactly maintain the chemicals under optimum spraying states.

Meanwhile, although portions relative to spraying of chemicals through at least one air transfer pipe 110 (see FIGS. 7 and 8), nozzle part cleaning using the cover part 100, and agitation of chemicals may be applied to the ultra-low volume spraying device according to the present embodiment, no description will be given of the configurations repeated with those of the above-mentioned embodiment.

Various embodiments have been described in the best mode for carrying out the invention. Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An ultra-low volume spraying device comprising:
a chemical container to receive chemicals therein;
a gas supply part to supply compressed gas;
a nozzle part to eject the chemicals mixed with the compressed gas;
a connection member comprising a first path to guide the compressed gas and a second path to guide the chemicals to the nozzle part while connecting the chemical container to the nozzle part; and
a cover part to selectively open and close an exit of at least one of the first and second paths,
wherein the second path is able to adjust an opening degree of the exit corresponding to characteristic information which exhibits characteristics of the chemicals.

2. The ultra-low volume spraying device according to claim 1, wherein the connection member comprises a connection part installed at a closure part of the chemical container and a connection pipe connected to the connection part so as to provide the first and second paths.

3. The ultra-low volume spraying device according to claim 2, wherein the closure part comprises therein a protrusion piece capable of adjusting a height thereof depending on characteristics of the chemicals and a fixing piece to fix a position of the protrusion piece.

4. The ultra-low volume spraying device according to claim 3, wherein the connection pipe comprises:
a liquid pipe communicating with the chemical container and serving as the second path accommodated within the first path;
an ejection control rod to adjust a sectional area of a liquid pipe ejection hole formed at an exit of the liquid pipe corresponding to the height of the protrusion piece; and
an elastic member to restrict movement of the ejection control rod.

5. The ultra-low volume spraying device according to claim 4, wherein:
the nozzle part is provided with a compressed gas ejection hole formed between the exit of the first path and the liquid pipe; and
the nozzle part is provided, at one end thereof, with a nozzle protrusion mounted to an exit side of the compressed gas ejection hole and liquid pipe ejection hole.

6. The ultra-low volume spraying device according to claim 5, wherein:
the cover part comprises a cover capable of opening and sealing the nozzle part by pivoting thereof and a cover driver to pivot the cover; and
when the cover seals the nozzle part, the cover comes into contact with the nozzle protrusion and the compressed gas ejected to the compress gas ejection hole is introduced into the liquid pipe ejection hole.

7. The ultra-low volume spraying device according to claim 3, wherein:
the closure part is mounted with a coupling protrusion, it being determined whether or not the coupling protrusion protrudes depending on characteristics of the chemicals received in the chemical container; and
the connection part comprises a switch to detect mounting of the coupling protrusion.

8. The ultra-low volume spraying device according to claim 3, wherein:
the closure part has an upper surface mounted with a magnetic substance, it being determined whether or not the magnetic substance exhibits magnetism depending on characteristics of the chemicals received in the chemical container; and
the connection part comprises a magnetic switch which is mounted to a contact surface coming into contact with the upper surface so as to detect mounting of the magnetic substance.

9. The ultra-low volume spraying device according to claim 3, wherein:
the closure part comprises a data storage part to store the characteristic information of the chemicals received in the chemical container; and
the connection part comprises a data reader to read the characteristic information of the chemicals stored in the data storage part.

10. The ultra-low volume spraying device according to any one of claims 7 to 9, further comprising a microcomputer to store the characteristic information of the chemicals provided from any one of the switch, magnetic switch, and data reader and sprayed information relative to actual spraying of the chemicals in a memory, and a communication part to transmit the information stored in the memory by wire or wireless communication.

11. The ultra-low volume spraying device according to claim 10, wherein the information stored in the memory is encoded so as to be decoded by a particular program, thereby being utilized as base data for an agricultural product traceability system or a farming daily record.

12. The ultra-low volume spraying device according to claim 1, wherein:
the gas supply part comprises a heater to apply heat and a compressed gas valve to adjust supply of the compressed gas; and
the compressed gas is at least one of carbon dioxide and air.

13. The ultra-low volume spraying device according to claim 1, further comprising an air transfer pipe to accommodate the nozzle part and a blower to move the chemicals ejected from the nozzle part.

14. The ultra-low volume spraying device according to claim 13, wherein:
the air transfer pipe is formed with air exits which are spaced apart from one another by a predetermined distance; and
the air exits are mounted with electrostatic generating electrodes, respectively.

15. The ultra-low volume spraying device according to claim 1, wherein the chemical container is coupled to or separated from the connection member.

16. An ultra-low volume spraying device comprising:
a chemical container to receive chemicals therein;
a gas supply part to supply compressed gas;
a nozzle part to eject the chemicals mixed with the compressed gas;
a connection member comprising a first path to guide the compressed gas and a second path to guide the chemicals to the nozzle part while connecting the chemical container to the nozzle part;
a data storage part to store characteristic information which exhibits characteristics of the chemicals received in the chemical container; and
a data reader to read the information of the data storage part,
wherein the second path is able to adjust an opening degree of an exit thereof corresponding to the read information.

17. The ultra-low volume spraying device according to claim 16, wherein the data storage part encodes and records the information.

18. The ultra-low volume spraying device according to claim 16, wherein the data storage part is a bar code type, and the data reader is a bar code reader.

19. The ultra-low volume spraying device according to claim 16, wherein the data storage part is a radio frequency identification (RFID) chip type, and the data reader is a RFID reader.

20. The ultra-low volume spraying device according to claim 16, wherein the connection member comprises a connection part installed at a closure part of the chemical container and a connection pipe providing the second path.

21. The ultra-low volume spraying device according to claim 20, further comprising a heater installed at the connection pipe so as to heat the chemicals guided by the second path.

22. The ultra-low volume spraying device according to claim 20, further comprising:
a valve seat installed within the connection pipe and extending from an exit of the second path; and
a valve seat adjuster to adjust a position of the valve seat depending on the read information.

23. The ultra-low volume spraying device according to claim 16, further comprising an eddy air generator installed at an outer side of the exit of the second path so as to generate eddy air using the compressed gas supplied through the first path, and a venturi tube to guide the eddy air and to form negative pressure, wherein the eddy air generator comprises a plurality of eddy air generators separately installed at front and rear sides of the venturi tube.

24. The ultra-low volume spraying device according to claim 16, wherein the data storage part is mounted to a closure part of the chemical container, and the data reader is mounted to the connection member.

25. The ultra-low volume spraying device according to claim 21, further comprising a microcomputer to control the heater based on the read information provided from the data reader.

26. The ultra-low volume spraying device according to claim 22, further comprising a microcomputer to control the valve seat adjuster based on the read information provided from the data reader.

27. The ultra-low volume spraying device according to claim 16, further comprising a microcomputer to store the characteristic information of the chemicals, which are received in the chemical container, provided from the data reader and sprayed information relative to actual spraying of the chemicals in a memory, and a communication part to transmit the information stored in the memory by wire or wireless communication.

28. The ultra-low volume spraying device according to claim 27, wherein the communication part comprises a universal serial bus (USB) interface which transmits information stored in the memory to a USB memory electrically connected to the microcomputer.

29. The ultra-low volume spraying device according to claim 27, wherein the information stored in the memory is encoded so as to be decoded by a particular program, thereby being utilized as base data for an agricultural product traceability system or a farming daily record.
